# EUROPEAN PATENT SPECIFICATION

(11) **EP 2 305 223 B1**
(45) Date of publication and mention of the grant of the patent: **11.09.2013**
(21) Application number: 09798192.2
(22) Date of filing: 30.03.2009
(51) Int. Cl.: A61K 31/115, A61P 43/00, A61P 17/02, A61P 19/08

(54) **AGENT FOR ACTIVATING STEM CELLS**
MITTEL ZUR AKTIVIERUNG VON STAMMZELLEN
PRÉPARATION VISANT À ACTIVER LES CELLULES SOUCHES

(30) Priority: 17.07.2008 RU 2008129554
(43) Date of publication of application: 06.04.2011
(73) Proprietor: Laskavy, Vladislav Nikolaevich, Saratov 410076 (RU); Goryunov, Dmitriy Vladimirovich, 8008 Zurich (CH); Ivanenko, Sergei Ivanovich, Moscow 123328 (RU)
(72) Inventor: Laskavy, Vladislav Nikolaevich, Saratov 410076 (RU); Goryunov, Dmitriy Vladimirovich, 8008 Zurich (CH); Ivanenko, Sergei Ivanovich, Moscow 123328 (RU)
(74) Representative: Hersina, Günter
(86) International application number: PCT/RU2009/000148
(87) International publication number: WO 2010/008317

(56) References cited:
- WO-A1-01/00200
- WO-A2-2010/032093
- FR-A1- 2 572 652
- RU-C1- 2 077 882
- RU-C2- 2 248 216
- US-A1- 2006 057 718
- MUKHERJEE SIDDHARTHA ET AL: "Pharmacologic targeting of a stem/progenitor population in vivo is associated with enhanced bone regeneration in mice", JOURNAL OF CLINICAL INVESTIGATION, vol. 118, no. 2, February 2008 (2008-02), pages 491-504, XP002671627, ISSN: 0021-9738
- ZHANG L ET AL: "Genotoxic effects of formaldehyde on human blood stem and progenitor cells", ENVIRONMENTAL AND MOLECULAR MUTAGENESIS; SPECIAL ISSUE: ABSTRACTS FROM THE ENVIRONMENTAL MUTAGEN SOCIETY 38TH ANNUAL MEETING, WILEY-LISS, INC. CHICHESTER, GB; ATLANTA, GEORGIA, USA, vol. 48, no. 7, 1 August 2007 (2007-08-01) , page 561, XP008149814, ISSN: 0893-6692, DOI: 10.1002/EM.20338 [retrieved on 2007-07-08]
- KATAYAMA N ET AL: "GROWTH AND FUNCTIONAL MODIFICATIONS IN FORMALDEHYDE-TREATED MOUSE BONE MARROW-DERIVED MAST CELLS", TOXICOLOGY IN VITRO, vol. 6, no. 3, 1992, pages 239-243, XP0025487834, ISSN: 0887-2333
- DATABASE CAPLUS [Online] CHEMICAL ABSTRACTS SERVICE, COLUMBUS, OHIO, US; 2005, Asaka, Takashi, Kikugawa, Hisao: "Effect of formaldehyde solution on fracture characteristics of bovine femoral compact bone", XP002671628, Database accession no. 2005:1088975 & ASAKA TAKASHI, KIKUGAWA, HISAO: "Effect of formaldehyde solution on fracture characteristics of bovine femoral compact bone.", NIPPON KINZOKU GAKKAISHI, vol. 69, no. 8, 2005, pages 711-714,
- 'Turning On Adult Stem Cells May Help Repair Bone' SCIENCE DAILY, [Online] 25 January 2008, page 1, XP008141221 Retrieved from the Internet: <URL:http://www.sciencedaily.com/releases/2 008/01/080124173809.htm> [retrieved on 2009-07-15]

## Description

The present invention relates to medicine, in particular - drugs designed to activate the body's own stem cells, as defined in the claims.

Human stem cells are used in modem medicine in treating many diseases, including cancer, cardiology and others. Stem cells can be derived from bone marrow and from umbilical cord blood and peripheral blood from embryonic tissues.

Stem cells are divided into mesenchymal (they are able to differentiate into cells of tissues of mesodermal origin), hematopoietic (precursors of blood cells), neuronal (progenitor cells of the nervous system) and others. Mesenchymal stem cells (MSCs) are often used as a graft, because they will enhance their own body reserves, promote the formation of cytokines and growth factors, MSCs are precursors of osteoblasts and promote the formation of remodeling units.

In the practice of cell therapy are widely distributed and used means of activation of stem cells, based on bringing in new organism of stem cells rather than activation of its own.

In particular, it is a means to activate the stem cells through glycoprotein (RF patent number 2,272,810 on Rd. IASC S07K16/28, pub. 27.03.2006), which contains as a glycoprotein polyclonal immunoglobulins of IgG class with an average molecular weight of 150 kDa, obtained both humoral immune response to complex antigens, CD34 + stem cells. This tool operates on the full range of antigenic determinants on the surface and inside of stem cells, which leads to their activation.

However, the tool is activated with their own stem cells. The process of obtaining the drug is durable, requires great effort.

It is known as a means to activate the stem cells (drug Filgastrim (Neupogen) firms F. Hoffmann-La Roche), is a glycoprotein consisting of 175 amino acids and having a molecular weight of 187-189 kilodaltons (kDa) obtained by expression of the human gene 5gdl-g31 in genome of E. coli. The product contains additional amino acids - methionine. Filgastrim shortens the maturation of neutrophils from progenitor cells with 5 to 1 day, speeds up the output of mature neutrophils from the bone marrow into the blood (B. Lord et al. Proc. Of the National Academy of Sci. Of USA, 1992, 1986, p.9499 -9503), enhances the chemotaxis of neutrophils (SPColgan et al., Experim.Hematology, 1992, 1920, p.1229-1234). It is used for standard cytotoxic chemotherapy, bone marrow transplantation, to mobilize the natural colony-stimulating factor in chronic neutropenia, to accelerate healing of wounds, etc.

However, the application of Filgastrim causes complications: bone pain, enlarged spleen, skin reactions, etc. Furthermore, the cost of treatment Filgastrim sufficiently high, U.S. $ 1500-3000.

Closest to the claimed a drug «bortesomib» (see www.rlsnet.ru / mnn_bortezomib.html), which is a modified-centered boric acid - a highly selective reversible inhibitor of 26S proteasome activity is present in the nucleus and the cytosol of all eukaryotic cells and catalyzes the splitting of the main proteins involved in the life cycle of cells. Chemical name - [(1R)-3-methyl-1[[(2S)-1-oxo-3-phenyl-2-[(pirasinilcarbonil) amino] propyl] amino] butyl], Brutto-formula: C₁₉H₂₅BN₄O₄.

*In vivo* «bortesomib» causes a slowdown in many experimental models of human tumors, including multiple myeloma. The drug is diluted with 0.9% NaCl to a concentration of 1 mg/ml, is administered intravenously. The main purpose of the drug - a multiple myeloma.

It was also established (sm.www.sciencedaily.com/releases/2008/01/080124 173,809. Htm), that «bortesomib» reduces the destruction of bone tissue, increases the activity of osteoblasts and bone formation, has a direct pharmacological effect on the mesenchymal stem cells. Thus, the drug activates stem cells in the body, reinforcing its reparative ability, in contrast to the widespread and widely used in the practice of cell therapy based on bringing in new organism of stem cells. Mukherjee et al. disclose in J. Clin. Invest., 118 (2). 2008, 491-504 that a tissue-resident adult stem cell population in vivo can be pharmacologically modified by bortezomib to promote a regenerative function in adult animals, such as bone formation.

The main drawback of this drug is its high toxicity and the many side effects: on the part of blood (thrombocytopenia, anemia), with the digestive system (nausea, vomiting, ileus, acute pancreatitis, hepatitis), the nervous system (headache, loss of consciousness, dysfunction of autonomic nervous system, convulsions), with Parties, the cardiovascular system (drop in blood pressure, congestive heart failure), etc.

The invention aims at solving the task of creating a non-toxic and not having side effects means to activate the body's own stem cells.

To solve this problem means to activate the stem cells containing the active principle of chemical origin, and sodium chloride for injection, according to the invention, it contains as an active formaldehyde in the following ratio, wt. %:

| | |
|---|---|
| Formaldehyde | 0.00003 - 0.004 |
| Sodium chloride for injection 0.85-0.95 % concentration | the rest. |

The present invention comprises the use of said composition for the manufacture of a medicament for the treatment of non-healing trophic ulcers and/or postoperative fistula and/or for restoration of bone tissue. Moreover the present invention comprises an *in vitro* method for activating a stem cell by administering said composition.

In the known authors of the sources of patent and scientific-technical information does not describe the non-toxic and do not have side-effects tools for the activation of stem cells, based not on infusing stem cells into the body, and the effects on the body's own cells - namely, on spleen cells and red bone marrow.

Known immunomodulatory agent containing formaldehyde and sodium chloride (see Russian patent number 2077882 on cells. A61K31/115 IPC, pub. 27/04/1997), the concentration of formaldehyde is 0.07-0.24 wt. %.

However, this drug does not activate the stem cells, but only has immunomodulatory effects.

The authors first identified the action of the drug concentration data (0.00003-0.004 wt. %) increasing aerobic oxidation, which in turn limits its effect on normal cells and leads to death of cancer cells.

This allows us to conclude that the presence of the claimed invention involves an "inventive step".

Aqueous solution of formaldehyde - a transparent colorless liquid with a peculiar pungent smell, mixed with water and alcohol in all proportions.

Formaldehyde - a representative of the class of aldehydes HCOH. Is a colorless gas with a pungent smell, they say. mass of 30.03, its density at 20 ° C is 0.815, melting point 92 ° C, the boiling temperature 19,2 ° C. It is soluble in water, alcohol. It is easy to polymerize, forming the polymerization in aqueous medium - paraformaldehyde, and in arid environments (butane, hexane) - POM.

Isotonic sodium chloride solution for injection - a colorless transparent liquid salty taste. The solution was sterile, pyrogen-free.

Sodium chloride - cubic crystals or white crystalline powder, salty taste, no smell. Soluble in water (1:3).

The claimed agent is a clear, colorless, odorless liquid is slightly salty taste.

The tool is prepared as follows.

Take a 37-40% solution of formaldehyde LIMITED Medicine, add it to the sterile 0.9-0.95 % solution of sodium chloride for injection prior to 0.00003-0.003 % solution of formaldehyde. Funds are held in a dark place at a temperature of 15-35 ° C.

### Example 1.

Take 0.01 ml of 37 % aqueous solution of formaldehyde health, add it to 99.99 ml of sterile 0.9 % aqueous (or 0.95 % strength) of isotonic sodium chloride solution. Mixture solutions are thoroughly mixed. The final concentration of formaldehyde in the funds received will be 0.0037 wt. %.

### Example 2.

Take 0.0001 ml of 37 % aqueous solution of formaldehyde health, add it to 99.9999 ml of sterile 0.9 % aqueous (or 0.95 % strength) of isotonic sodium chloride solution. Mixture solutions are thoroughly mixed. The final concentration of formaldehyde in the funds received will be equal to 0.000037 wt. %.

### Example 3.

Take 0.01 ml of 40 % aqueous medicinal solution of formaldehyde. The tool is prepared in accordance with the above in Example 2. The final concentration of formaldehyde will be equal to 0.004 wt. %.

### Example 4.

Take 0.0001 ml 40 % aqueous solution of formaldehyde health; add it to 99.9999 ml of sterile 0.95 % aqueous isotonic sodium chloride solution. Mixture solutions are thoroughly mixed. The final concentration of formaldehyde in the funds received will be equal to 0.00003 wt. %.

To prove the activation of stem cells carried out a study on the proliferation of drug action and toxicity in spleen cells and bone marrow.

The experiments used 3-month-old mice of line C57B1 / 6 (males). Mice were killed by decapitation, under sterile conditions, were extracted spleen and red bone marrow. Fragments of spleen were homogenized thoroughly in a glass homogenizer in medium 199, filtered through sterile gauze and washed 3 times in medium 199 for 5 minutes at 1500 rpm.

Bone marrow cells were extracted with a syringe and the environment 199 of the femur. They were then carefully pipetted and washed with the above method.

After this, splenocytes and bone marrow cariotsites transferred to complete culture medium (medium RPMJ-1640 +10 % FCS (fetal calf serum) + 2 mM L-glutamine + 25 mM Hepes-buffer + 2 + mercaptoethanol 4g/ml gentamicin) and adjusted to 1h106 concentration of nucleated cells per 1 ml. After the above mentioned procedures, the number of viable cells was 93-98 %.

Cell culturing was performed under sterile conditions in a humid atmosphere with 5% CO2. Number of live and dead cells was evaluated using a fluorescence microscope after adding to the cell suspension mixture of solutions of acridine orange (0.25 mg/ml) and ethidium bromide (0.25 mg/ml). Proliferative activity of cells was assessed after adding to them at 4:00 (after incubation) 1 mkKYu ³H-thymidine (sp. activity).

At the same cells after incubation with thymidine were transferred to glass fiber filters, washed with a large excess of water, recorded 96o ethanol and then dried in air. After that, filters were immersed in standard toluene scintillator-based POP and POPOP (3 samples per dilution of the drug). Radioactivity of samples was calculated using β-counter (the number of β-decay of 3H-thymidine in 1 minute).

In cell culture drug was added at different concentrations and take into account the percentage of dead cells after 24 hours incubation.

Results to determine the toxicity of the drug in different concentrations are presented in Table 1.

From Table 1 that different concentrations of the drug (0.0035-0.0000273) as when added to splenocytes and cariotsytes, increase the number of dead cells compared with control in 1.4-2.5 times.

The result of studying the proliferative capacity of splenocytes and cariotsytes presented in Table 2.

Table 2 shows that the proliferation of splenocytes compared to control increases in 1.5-4.2 times, and kariotsitov from 2,1 to 12.7 times.

We studied the proliferative activity of splenocytes in a comparative aspect after 24 and 44 hours of incubation. In this case, proliferation activity was estimated by the number of pulses per minute of 3H-thymidine injected into the cell culture for 4 hours. The results are presented in Table 3.

From Table 3 shows that at all concentrations of the drug cell proliferation increases with the incubation period compared with the control at 1.5 - 14 times.

These results indicate that the drug in vitro enhances the proliferation of spleen cells and bone marrow. In this case, the partial cell death is not determinative in the process of reproduction and does not affect the enhancement of proliferation of the remaining normal cells.

The drug has been tried out in the working concentration on a limited number of patients: from non-healing trophic ulcers, postoperative fistula, at the turn.

The drug was injected intramuscularly 1 time per week for 1-2 months.

### Example 1.

Patient N., aged 35, with postoperative fistula.

Following a course of treatment for 2 months there was a full healing of wounds without re-operative intervention.

### Example 2.

Patient S., 1972 - incurable trophic ulcer.

After the course within 1 month occurred purification and complete healing of ulcers.

### Example 3.

Patient K., aged 45 - fracture of the ankle joint.

After treatment for 1.5 months, there was a restoration of bone tissue without any complications. Radiologically proven accelerated the recovery process.

Thus, the claimed means of *in vitro* and applied to a limited number of patients demonstrates the possibility of its use to enhance their own stem cells.

### The percentage of dead cells (splenocytes and bone marrow cells) 3-month-old mice S57VL / 6 after 24 h incubation with different concentrations of the drug.

| Cells | Drug concentration | | | | | | | Control |
|---|---|---|---|---|---|---|---|---|
| | 0.0035 | 0.00175 | 0.000875 | 0.00044 | 0.00011 | 0.000055 | 0.0000273 | |
| Splenocytes | 65 | 65 | 65 | 73 | - | 76 | 45 | 31 |
| Cells bone brain | 51 | 66 | 57 | 64 | 69 | 56 | - | 33 |

### Proliferation of splenocytes and bone marrow cells after 24 h incubation with different concentrations of the drug.

| Cells | Drug concentration | | | | | | | Control |
|---|---|---|---|---|---|---|---|---|
| | 0.0035 | 0.00175 | 0.000875 | 0.00044 | 0.00011 | 0.000055 | 0.0000273 | |
| Splenocytes | 451 | 770 | 1750 | 2439 | - | 5050 | 4000 | 1200 |
| Cells bone brain | 6000 | 9000 | 4400 | 3300 | 1500 | 700 | - | 711 |

### Proliferative activity of splenocytes 100,000 3-month mouse S57VL / 6 after 24 and 44 h incubation with different concentrations of the drug.

| Term Incubation | Drug concentration | | | | | | | Control |
|---|---|---|---|---|---|---|---|---|
| | 0.0035 | 0.00175 | 0.000875 | 0.00044 | 0.00011 | 0.00005 | 0.0000273 | |
| 24 hours | 451 | 770 | 1750 | 2439 | - | 5050 | 4000 | 1200 |
| 44 hours | 2692 | 6229 | 9964 | 25528 | 13247 | 12756 | 9930 | 1826 |

## Claims

1. Use of an active ingredient and sodium chloride for injection for the manufacture of a medicament for the treatment of non-healing trophic ulcers and/or postoperative fistula and/or for restoration of bone tissue, **characterized in that** as the active ingredient the medicament contains formaldehyde in the following ratio (wt. %):
| | |
|---|---|
| Formaldehyde | 0.00003-0.004 |
| Sodium chloride for injection 0.85-0.95 % concentration | the rest. |

2. Method for activating a stem cell *in vitro* comprising administering a composition containing an active ingredient and sodium chloride for injection, wherein as the active ingredient the composition contains formaldehyde in the following ratio (wt. %):
| | |
|---|---|
| Formaldehyde | 0.00003-0.004 |
| Sodium chloride for injection 0.85-0.95 % concentration | the rest. |

## Patentansprüche

1. Verwendung eines Wirkstoffs und von Natriumchlorid zur Injektion für die Herstellung eines Medikaments zur Behandlung von nicht-heilenden trophischen Ulzera und/oder postoperativer Fistel und/oder zur Wiederherstellung von Knochengewebe, **dadurch gekennzeichnet, dass** das Medikament Formaldehyd als den Wirkstoff im folgenden Verhältnis (Gew.-%) enthält:
| | |
|---|---|
| Formaldehyd | 0,00003 bis 0,004 |
| Natriumchlorid zur Injektion, Konzentration 0,85 bis 0,95 % | Rest. |

2. Verfahren zum Aktivieren einer Stammzelle *in vitro*, umfassend das Verabreichen einer Zusammensetzung, die einen Wirkstoff und Natriumchlorid zur Injektion enthält, wobei die Zusammensetzung Formaldehyd als den Wirkstoff im folgenden Verhältnis (Gew.-%) enthält:
| | |
|---|---|
| Formaldehyd | 0,00003 bis 0,004 |
| Natriumchlorid zur Injektion, Konzentration 0,85 bis 0,95 % | Rest. |

## Revendications

1. Utilisation d'un ingrédient actif et de chlorure de sodium pour injection pour la fabrication d'un médicament destiné au traitement d'ulcères trophiques persistants et/ou d'une fistule postopératoire et/ou destiné à la restauration d'un tissu osseux, **caractérisée en ce qu'**en tant qu'ingrédient actif, le médicament contient du formaldéhyde selon le rapport (% en poids) suivant :
| | |
|---|---|
| formaldéhyde | 0,00003 à 0,004 |
| chlorure de sodium pour injection à une concentration de 0,85 à 0,95 % | le reste. |

2. Méthode d'activation d'une cellule souche *in vitro* comprenant l'administration d'une composition contenant un ingrédient actif et du chlorure de sodium pour injection, dans laquelle en tant qu'ingrédient actif, la composition contient du formaldéhyde selon le rapport (% en poids) suivant :
| | |
|---|---|
| formaldéhyde | 0,00003 à 0,004 |
| chlorure de sodium pour injection à une concentration de 0,85 à 0,95 % | le reste. |
